# EUROPEAN PATENT APPLICATION

(11) **EP 4 516 284 A1**
(43) Date of publication of application: **05.03.2025**
(21) Application number: 23795832.7
(22) Date of filing: 01.02.2023
(51) Int. Cl.: A61K 8/81, A61K 8/73, A61K 8/84, A61K 8/86

(54) **COSMETIC**

(30) Priority: 28.04.2022 JP 2022075148
(71) Applicant: Shiseido Company, Ltd., Chuo-ku Tokyo 104-0061 (JP)
(72) Inventor: KANEKO, Ryosuke, Tokyo 104-0061 (JP); MATSUO, Ayano, Tokyo 104-0061 (JP); SOGABE, Atsushi, Tokyo 104-0061 (JP); OHTANI, Tsuyoshi, Tokyo 104-0061 (JP)
(74) Representative: Ter Meer Steinmeister & Partner
(86) International application number: PCT/JP2023/003247
(87) International publication number: WO 2023/210084

(57) **Abstract**

Provided is a cosmetic suitable for high-temperature low-humidity environments.

A cosmetic containing a functional polymer in which a temperature-responsive polymer and a hydrophilic polymer form an interpenetrating polymer network structure or a semi-interpenetrating polymer network structure.

## Description

### FIELD

The present invention relates to a cosmetic.

### BACKGROUND

Due to climate change or humans themselves becoming more active, humans are increasingly living in harsh environments. Therefore, there is a need to protect the skin with cosmetics suitable for such harsh environments.

For example, it is known that Japan experiences high temperatures and high humidity in the summer season. In contrast, the Middle East and continental inland regions experience high temperatures and low humidity in the summer period. Thus, cosmetics suitable for high-temperature low-humidity environments are necessary.

Although a large number of cosmetics suitable for the environment of Japan, which has high temperatures and high humidity in the summer period and low temperatures and low humidity in the winter season, have been developed, cosmetics suitable for the high-temperature low-humidity environments stated above have not been sufficiently investigated, and the need therefor remains.

PTL 1 discloses a moisture absorbing material comprising: a dried product of a polymer gel, the polymer gel including a stimuli-responsive polymer whose affinity with water changes reversibly in response to an external stimulus and a hydrophilic polymer, and the stimuli-responsive polymer and the hydrophilic polymer forming an interpenetrating polymer network structure or a semi-interpenetrating polymer network structure.

### [CITATION LIST]

### [PATENT LITERATURE]

[PTL 1] WO 2016/068129

### SUMMARY

### [TECHNICAL PROBLEM]

The present invention aims to improve the above circumstances, and an object thereof is to provide a cosmetic suitable for high-temperature low-humidity environments.

### [SOLUTION TO PROBLEM]

The present invention for achieving the above object is as follows.

### <Aspect 1>

A cosmetic comprising a functional polymer in which a temperature-responsive polymer and a hydrophilic polymer form an interpenetrating polymer network structure or a semi-interpenetrating polymer network structure.

### <Aspect 2>

The cosmetic according to Aspect 1, wherein the functional polymer has a first water content ratio at a temperature higher than a predetermined temperature that is smaller than a second water content ratio at a temperature lower than the predetermined temperature, and the functional polymer thereby releases, in an atmosphere having a temperature higher than the predetermined temperature, moisture absorbed in an atmosphere having a temperature lower than the predetermined temperature.

### <Aspect 3>

The cosmetic according to Aspect 2, wherein the predetermined temperature is any temperature in a range of 5 to 60°C.

### <Aspect 4>

The cosmetic according to any one of Aspects 1 to 3, wherein the temperature-responsive polymer is at least one polymer selected from the group consisting of poly(N-alkyl (meth)acrylamide); poly(N-vinylalkylamide); poly(N-vinylpyrrolidone); poly(N-vinylcaprolactam); poly(2-alkyl-2-oxazoline); polyvinyl alkyl ether; polyethylene oxide and polypropylene oxide and copolymers thereof; copolymers of glycols such as polyethylene glycol and polypropylene glycol and diisocyanates such as hexamethylene diisocyanate, diphenylmethane diisocyanate, toluene diisocyanate, and methylene diphenyl diisocyanate; copolymers composed of components selected from alkyl (meth)acrylates such as methyl (meth)acrylate, ethyl (meth)acrylate, and butyl (meth)acrylate, acrylamides such as N-phenyl acrylamide, isopropyl acrylamide, and N-tert-butyl acrylamide, and di(meth)acrylic acids such as 1,3-butandiol dimethacrylate, 1,6-hexanediol dimethacrylate, and PEG di(meth)acrylate; poly(oxyethylene vinyl ether); cellulose derivatives; polyurethane derivatives; and copolymers comprising these, or a crosslinked body thereof.

### <Aspect 5>

The cosmetic according to any one of Aspects 1 to 4, wherein the hydrophilic polymer is at least one polymer selected from the group consisting of alginic acid, hyaluronic acid, carrageenan, xanthan gum, glucomannan, tara gum, locust bean gum, guar gum, fenugreek gum, chitosan, cellulose derivatives, poly(meth)acrylic acid, polyethylene glycol, and copolymers thereof, or a crosslinked body thereof.

### <Aspect 6>

The cosmetic according to any one of Aspects 1 to 5, which is a powder cosmetic.

### <Aspect 7>

The cosmetic according to any one of Aspects 1 to 5, which is a liquid cosmetic.

### [ADVANTAGEOUS EFFECTS OF INVENTION]

According to the present invention, a cosmetic suitable for high-temperature low-humidity environments can be provided.

### BRIEF DESCRIPTION OF DRAWINGS

FIG. 1 is a diagram showing water absorption amounts in Example 1.
FIG. 2 is a diagram showing water absorption amounts in Comparative Example 1.
FIG. 3 is a diagram showing water release amounts in Example 1.
FIG. 4 is a diagram showing water release amounts in Comparative Example 1.
FIG. 5 is a diagram showing water absorption amounts in Example 2.
FIG. 6 is a diagram showing water release amounts in Example 2.
FIG. 7 consists of diagrams showing surface moisture content of the skin in each of Examples 3 and 4 and Comparative Examples 2 and 3.
FIG. 8 consists of diagrams showing surface moisture content of the skin in each of Example 5 and Comparative Example 4.

### DESCRIPTION OF EMBODIMENTS

Hereinafter, the embodiments of the present invention will be described in detail. Note that, the present invention is not limited to the following embodiments, and various modifications can be made within the scope of the invention.

### <<Cosmetic>>

The cosmetic of the present invention is
a cosmetic comprising a functional polymer in which a temperature-responsive polymer and a hydrophilic polymer form an interpenetrating polymer network structure and a semi-interpenetrating polymer network structure.

The cosmetic of the present invention contains a functional polymer having the above unique properties, whereby the polymer can release water to supply moisture to the skin in the outdoors at high temperature and low humidity. In an air-conditioned room with cooling, the polymer can absorb water in preparation to release water outdoors. That is, the cosmetic of the present invention is a cosmetic suitable for high-temperature low-humidity environments.

Particularly, in the cosmetic of the present invention, the functional polymer has a first water content ratio at a temperature higher than a predetermined temperature that is smaller than a second water content ratio at a temperature lower than the predetermined temperature, and thereby the polymer releases, in an atmosphere having a temperature higher than the predetermined temperature, moisture absorbed in an atmosphere having a temperature lower than the predetermined temperature.

The first water content ratio and the second water content ratio of the functional polymer, in each case, refer to a proportion of moisture relative to a dry weight of the functional polymer (unit: g-moisture/g-dry mass). In the present invention, the specific values (unit: g/g-dry mass) of the second water content ratio of the functional polymer are not particularly limited, and each independently may be greater than 0.0, 0.5 or greater, 1.0 or greater, 1.1 or greater, 1.5 or greater, 2.0 or greater, 2.5 or greater, 3.0 or greater, 3.5 or greater, 4.0 or greater, 4.5 or greater, 5.0 or greater, 5.5 or greater, 6.0 or greater, 6.5 or greater, 7.0 or greater, 7.5 or greater, 8.0 or greater, 8.5 or greater, 9.0 or greater, 9.5 or greater, or 10 or greater, and may be 30 or less, 25 or less, or 20 or less.

In the present invention, the first water content ratio of the functional polymer needs only to be smaller than the second water content ratio. For example, the first water content ratio of the functional polymer relative to the second water content ratio may be less than 1.0, 0.9 or less, 0.8 or less, 0.7 or less, 0.6 or less, 0.5 or less, 0.4 or less, 0.3 or less, 0.2 or less, or 0.1 or less.

The predetermined temperature is not particularly limited, and for example, may be any temperature at 5°C or higher, 10°C or higher, 15°C or higher, 20°C or higher, 25°C or higher, 30°C or higher, 35°C or higher, or 40°C or higher, and may be any temperature at 60°C or lower, 55°C or lower, or 50°C or lower.

In the functional polymer contained in the cosmetic of the present invention, a temperature-responsive polymer and a hydrophilic polymer form an interpenetrating polymer network structure or a semi-interpenetrating polymer network structure.

The "interpenetrating polymer network structure" herein refers to a structure in which different polymers, all of which are crosslinked polymers, are mutually entangled in a state where the crosslinked networks of the individual polymers are not chemically bonded and exist independently. The "semi-interpenetrating polymer network structure" refers to a structure in which different polymers, one of which is a crosslinked polymer and the other a linear polymer, are mutually entangled in a state where the individual polymers are not chemically bonded and exist independently.

In the case of an interpenetrating polymer network structure, the temperature-responsive polymer and the hydrophilic polymer are both crosslinked polymers each having a crosslinked network, and the crosslinked network of the temperature-responsive polymer and the crosslinked network of the hydrophilic polymer form a mutually entangled structure without being chemically bonded, i.e., an interpenetrating polymer network structure.

In the case of a semi-interpenetrating polymer network structure, either the temperature-responsive polymer or the hydrophilic polymer is a crosslinked polymer having a crosslinked network, the other is a linear polymer, and the temperature-responsive polymer and the hydrophilic polymer form a mutually entangled structure without being chemically bonded, i.e., a semi-interpenetrating polymer network structure.

In the present invention, the proportions of the temperature-responsive polymer and the hydrophilic polymer contained in the functional polymer are not particularly limited. For example, the hydrophilic polymer relative to the temperature-responsive polymer, in proportion of weight excluding the weight of the crosslinking agent described below, may be 5.0% by mass or greater, 10% by mass or greater, 15% by mass or greater, or 20% by mass or greater, and may be 1000% by mass or less, 700% by mass or less, or 500% by mass or less.

The functional polymer content in the cosmetic of the present invention is not particularly limited, and for example, may be 0.01% by mass or greater, 0.1% by mass or greater, 0.5% by mass or greater, 1.0% by mass or greater, 3.0% by mass or greater, 5.0% by mass or greater, 7.0% by mass or greater, or 10% by mass or greater relative to the entirety of the cosmetic. Of these, 0.1% by mass or greater is preferable, and 0.5% by mass or greater is more preferable. The upper limit of the content of the functional polymer is not particularly limited, and for example, may be 100% by mass or less, 80% by mass or less, 50% by mass or less, 20% by mass or less, or 10% by mass or less relative to the entirety of the cosmetic.

### (Temperature-responsive polymer)

In the present invention, the temperature-responsive polymer needs only to be a polymer with a lower critical solution temperature (LCST; hereinafter, also referred to as "LCST") and is not particularly limited. The polymer with a LCST is hydrophilic at low temperature, but becomes hydrophobic at the LCST or higher. The LCST herein refers to a boundary temperature at which a polymer, when dissolved in water, is hydrophilic and soluble in water at low temperature, but is hydrophobic and insoluble at a certain temperature or higher. The LCST of the temperature-responsive polymer can be measured by, for example, the method used in the Examples.

The predetermined temperature of the functional polymer described above may be the LCST of the temperature-responsive polymer, or may be a temperature value within a certain range from such a LCST. Specifically, the absolute value of the difference (predetermined temperature - LCST) between the predetermined temperature of the functional polymer and the LCST may be 0°C or higher, 5°C or higher, 10°C or higher, 15°C or higher, or 20°C or higher, or may be 40°C or lower or 35°C or lower.

In the present invention, the temperature-responsive polymer is not particularly limited. More specifically, the temperature-responsive polymer, for example, may be at least one polymer selected from poly(N-alkyl (meth)acrylamide) such as poly(N-isopropyl (meth)acrylamide), poly(N-normalpropyl (meth)acrylamide), poly(N-methyl (meth)acrylamide), poly(N-ethyl (meth)acrylamide), poly(N,N-diethyl (meth)acrylamide), poly(N-normalbutyl (meth)acrylamide), poly(N-isobutyl (meth)acrylamide), and poly(N-t-butyl (meth)acrylamide); poly(N-vinylalkylamide) such as poly(N-vinylisopropylamide), poly(N-vinylnormalpropylamide), poly(N-vinylnormalbutylamide), poly(N-vinylisobutylamide), and poly(N-vinyl-t-butylamide); poly(meth)acrylic acid ester such as poly(oligoethylene glycol (meth)acrylate), poly(dimethylaminoethyl (meth)acrylate), and poly(hydroxypropyl (meth)acrylate); poly(N-vinylpyrrolidone); N-vinylcaprolactam; poly(2-alkyl-2-oxazoline) such as poly(2-ethyl-2-oxazoline), poly(2-isopropyl-2-oxazoline), and poly(2-normalpropyl-2-oxazoline); polyvinyl alkyl ether such as polyvinyl methyl ether and polyvinyl ethyl ether; polyethylene oxide, polypropylene oxide, and copolymers thereof; copolymers of glycols such as polyethylene glycol and polypropylene glycol and diisocyanates such as hexamethylene diisocyanate, diphenylmethane diisocyanate, toluene diisocyanate, and methylene diphenyl diisocyanate; copolymers consisting of components selected from alkyl (meth)acrylates such as methyl (meth)acrylate, ethyl (meth)acrylate, and butyl (meth)acrylate, acrylamides such as N-phenyl acrylamide, isopropyl acrylamide, and N-tert-butyl acrylamide, and di(meth)acrylic acids such as 1,3-butanediol dimethacrylate, 1,6-hexanediol dimethacrylate, and PEG di(meth)acrylate; poly(oxyethylene vinyl ether); cellulose derivatives such as methyl cellulose, ethyl cellulose, hydroxypropyl cellulose, and hydroxypropyl methyl cellulose; polyurethane derivatives; and copolymers comprising these, or a crosslinked body thereof.

In order to form an interpenetrating polymer network structure or a semi-interpenetrating polymer network structure with a temperature-responsive polymer and a hydrophilic polymer, at least the temperature-responsive polymer or the hydrophilic polymer is a crosslinked body in the present invention.

Temperature-responsive polymers as crosslinked bodies include polymers obtained by polymerizing one or more monomers in the presence of a crosslinking agent.

As such monomers, for example, monomers such as vinyl alkyl ethers, such as vinyl methyl ether and vinyl ethyl ether; ethylene oxide and propylene oxide; 2-alkyl-2-oxazolines, such as 2-ethyl-2-oxazoline, 2-isopropyl-2-oxazoline, and 2-normalpropyl-2-oxazoline; N-alkyl (meth)acrylamides, such as N-isopropyl (meth)acrylamide, N-normalpropyl (meth)acrylamide, N-methyl (meth)acrylamide, N-ethyl (meth)acrylamide, N,N-diethyl (meth)acrylamide, N-normalbutyl (meth)acrylamide, N-isobutyl (meth)acrylamide, and N-t-butyl (meth)acrylamide; N-vinylalkylamides, such as N-vinylisopropylamide, N-vinylnormalpropylamide, N-vinylnormalbutylamide, N-vinylisobutylamide, and N-vinyl-t-butylamide; (meth)acrylic acid esters, such as oligoethylene glycol (meth)acrylate, dimethylaminoethyl (meth)acrylate, and hydroxypropyl (meth)acrylate; N-vinylpyrrolidone; and N-vinylcaprolactam, or combinations of two or more of these monomers can be used.

The above crosslinking agent is not particularly limited and includes ones generally used as crosslinking agents. For example, metal ions such as calcium ion and zinc ion; crosslinkable monomers having a polymerizable functional group, such as ethylene glycol di(meth)acrylate, propylene glycol di(meth)acrylate, N,N'-methylenebis(meth)acrylamide, tolylene diisocyanate, divinylbenzene, polyethylene glycol di(meth)acrylate, polypropylene glycol di(meth)acrylate, glycerol di(meth)acrylate, 1,6-hexanediol di(meth)acrylate, 1,9-nonanediol di(meth)acrylate, 1,10-decanediol di(meth)acrylate, and neopentylene glycol diacrylate; glutaraldehyde; polyhydric alcohols; polyamines; polycarboxylic acids; diamines such as ethylenediamine, hexamethylenediamine, and phenylenediamine; acid anhydrides such as pyromellitic anhydride, ethylenediaminetetraacetic dianhydride, diethylenetriaminepentaacetic dianhydride, 1,2,4,5-cyclohexanetetracarboxylic dianhydride, and 1,2,3,4-cyclopentanetetracarboxylic dianhydride; dicarboxylic acids such as maleic acid, succinic acid, sebacic acid, adipic acid, terephthalic acid, fumaric acid, and isophthalic acid; aromatic dithiols such as 1,4-benzenedithiol, 1,3-benzenedithiol, 1,3,5-benzenetrithiol, 4,4'-diphenyldithiol, 4,4'-thiobisbenzenethiol, and 1,5-dimercaptonaphthalene; and aromatic diols such as catechol, resorcinol, hydroquinone, catecholamine, pyrogallol, catechin, and urushiol can be suitably used. These crosslinking agents may be used independently, or may be used in combinations of two or more.

The temperature-responsive polymer as a crosslinked body can be obtained by reacting an uncrosslinked temperature-responsive polymer, for example, a temperature-responsive polymer exemplified above, with a crosslinking agent described above to form a network structure.

The molecular weight of the temperature-responsive polymer is not particularly limited, and for example, is a number average molecular weight of preferably 2000 or greater, and more preferably 3000 or greater, determined by gel permeation chromatography (GPC).

### (Hydrophilic polymer)

In the present invention, the hydrophilic polymer needs only to be a hydrophilic polymer generally used in cosmetics and is not particularly limited. Examples thereof can include polymers having a hydrophilic group such as a hydroxyl group, a carboxyl group, a sulfonate group, a phosphate group, or an amino group on a side chain or main chain. More specific examples of the hydrophilic polymer can include poly(meth)acrylate, polymaleic acid, polyvinyl sulfonic acid, polyvinylbenzene sulfonate, polyacrylamide alkyl sulfonate, polydimethylaminopropyl (meth)acrylamide, copolymers thereof with any of (meth)acrylamide, hydroxyethyl (meth)acrylate, and (meth)acrylic acid alkyl esters; complex of polydiemthylaminopropyl (meth)acrylamide and polyvinyl alcohol, complex of polyvinyl alcohol and poly(meth)acrylic acid; poly(meth)acrylonitrile, polyallylamine, polyvinyl alcohol, polyethylene glycol, polypropylene glycol, poly(meth)acrylamide, poly-N,N'-dimethyl(meth)acrylamide, poly-2-hydroxyethyl methacrylate, poly-alkyl(meth)acrylate, polydimethylaminopropyl (meth)acrylamide, poly(meth)acrylonitrile, and copolymers of the above polymers; polysaccharides such as alginic acid, hyaluronic acid, carrageenan, xanthan gum, glucomannan, tara gum, locust bean gum, guar gum, and fenugreek gum; chitosan; and cellulose derivatives such as carboxymethyl cellulose, methyl cellulose, ethyl cellulose, and hydroxyethyl cellulose. It is more preferable that the hydrophilic polymer be a crosslinked body of any of these hydrophilic polymers.

As monomers constituting the hydrophilic polymer as a crosslinked body, for example, monomers such as (meth)acrylic acid, allylamine, vinyl acetate, (meth)acrylamide, N,N'-dimethyl (meth)acrylamide, 2-hydroxyethyl methacrylate, alkyl (meth)acrylate, maleic acid, vinyl sulfonate, vinylbenzene sulfonate, acrylamide alkyl sulfonate, dimethylaminopropyl (meth)acrylamide, and (meth)acrylonitrile or combinations of two or more of these monomers can be used.

The above crosslinking agent is not particularly limited and includes ones generally used as crosslinking agents. For example, metal ions such as calcium ion and zinc ion; crosslinkable monomers having a polymerizable functional group, such as ethylene glycol di(meth)acrylate, propylene glycol di(meth)acrylate, N,N'-methylenebis(meth)acrylamide, tolylene diisocyanate, divinylbenzene, and polyethylene glycol di(meth)acrylate; glutaraldehyde; polyhydric alcohols; polyamines; and polycarboxylic acids can be suitably used. These crosslinking agents may be used independently, or may be used in combinations of two or more.

When the temperature-responsive polymer is a crosslinked product, the crosslinked product may be a crosslinked body obtained by reacting an uncrosslinked hydrophilic polymer, for example, a polymer obtained by polymerizing monomers; a polysaccharide such as alginic acid, hyaluronic acid, carrageenan, xanthan gum, glucomannan, tara gum, locust bean gum, guar gum, or fenugreek gum; chitosan; or a cellulose derivative such as carboxymethyl cellulose, methyl cellulose, ethyl cellulose, or hydroxyethyl cellulose, with a crosslinking agent to form a network structure.

The molecular weight of the hydrophilic polymer is not particularly limited, and for example, is a number average molecular weight of preferably 2000 or greater, and more preferably 3000 or greater, determined by GPC.

### <Form of cosmetic>

The form of the cosmetic of the present invention is not particularly limited, and for example, may be in any form such as a powder cosmetic, a liquid cosmetic, a gel cosmetic, a cream cosmetic, a balm cosmetic, an aerosol cosmetic, a mist cosmetic, or a foam cosmetic. The cosmetic of the present invention may further contain one or more components other than the functional polymer as appropriate according to these forms, and various components can be appropriately blended within ranges that do not affect the effect of the present disclosure. Examples of such optional components include water-phase components, oil-phase components, and powder components. More specifically, examples of optional components can include humectants, emollients, thickeners, water-soluble polymers, oil-soluble polymers, film-forming agents such as siliconated polysaccharides, higher fatty acids such as isostearic acid, metal-ion sequestrants, lower alcohols such as ethanol, higher alcohols such as stearyl alcohol, polyhydric alcohols such as 1,3-butylene glycol, various extracts, saccharides, amino acids, organic amines, chelating agents, ultraviolet absorbers, pH modifiers, skin nutrients, vitamins; water-soluble agents applicable to drugs, quasi-drugs, and cosmetics; buffering agents, anti-fading agents, preservatives, propellants, organic powders, pigments, dyes, colorants, and perfumes. The optional components may be appropriately blended independently or in combinations of two or more, and are not limited thereto.

As water-phase components, water, water-soluble alcohols, and thickeners that are generally used in cosmetics and quasi-drugs can be blended, and humectants, chelating agents, preservatives, and colorants can further be appropriately blended as desired.

The water contained in the cosmetic of the present invention is not particularly limited, and for example, includes purified water, ion-exchanged water, and tap water.

Examples of water-soluble alcohols include lower alcohols, polyhydric alcohols, polyhydric alcohol polymers, dihydric alcohol alkyl ethers, divalent alcohol alkyl ethers, dihydric alcohol ether esters, glycerin monoalkyl ether, sugar alcohols, monosaccharides, oligosaccharides, polysaccharide, and derivatives thereof.

Examples of lower alcohols include ethanol, propanol, isopropanol, isobutyl alcohol, and t-butyl alcohol.

Examples of polyhydric alcohols include, but are not limited to, dihydric alcohols (for example, dipropylene glycol, 1,3-buylene glycol, ethylene glycol, trimethylene glycol, 1,2-butylene glycol, tetramethylene glycol, 2,3-butylene glycol, pentamethylene glycol, 2-butene-1,4-diol, hexylene glycol, and octylene glycol); trihydric alcohols (for example, glycerin and trimethylolpropane); tetrahydric alcohols (for example, pentaerythritols such as diglycerin and 1,2,6-hexanetriol); pentahydric alcohols (for example, xylitol and triglyercin); hexahydric alcohols (for example, sorbitol and mannitol); polyhydric alcohol polymers (for example, diethylene glycol, dipropylene glycol - triethylene glycol, polypropylene glycol, tetraethylene glycol, diglycerin - triglycerin, tetraglycerin, and polyglycerin); dihydric alcohol alkyl ethers (for example, ethylene glycol monomethyl ether, ethylene glycol monoethyl ether, ethylene glycol monobutyl ether, ethylene glycol monophenyl ether, ethylene glycol monohexyl ether, ethylene glycol mono 2-methylhexyl ether, ethylene glycol isoamyl ether, ethylene glycol benzyl ether, ethylene glycol isopropyl ether, ethylene glycol dimethyl ether, ethylene glycol diethyl ether, and ethylene glycol dibutyl ether); divalent alcohol alkyl ethers (for example, diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether triethylene glycol monomethyl ether triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether); dihydric alcohol ether esters (for example, ethylene glycol monomethyl ether acetate, ethylene glycol monoethyl ether acetate, ethylene glycol monobutyl ether acetate, ethylene glycol monophenyl ether acetate, ethylene glycol diadivate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate); glycerin monoalkyl ethers (for example, xyl alcohol, selachyl alcohol, and batyl alcohol); sugar alcohols (for example, maltotriose, mannitol, sucrose, erythritol, glucose, fructose, starch degradation sugars, maltose, and starch degradation sugar-reducing alcohols); Glysolid; tetrahydrofurfuryl alcohol; POE-tetrahydrofurfuryl alcohol; POP-butyl ether; POP/POE-butyl ether; tripolyoxypropylene glycerin ether; POP-glycerin ether; POP-glycerin ether phosphate; POP/POE-pentaerythritol ether; and polyglycerin.

Examples of monosaccharides include trioses (for example, D-glyceraldehyde and dihydroxyacetone); tetroses (for example, D-erythrose, D-erythrulose, D-threose, and erythritol); pentoses (for example, L-arabinose, D-xylose, L-lyxose, D-arabinose, D-ribose, D-ribulose, D-xylulose, and L-xylulose); hexoses (for example, D-glucose, D-talose, D-psicose, D-galactose, D-fructose, L-galactose, L-mannose, and D-tagatose); heptoses (for example, aldoheptose and heplose); octoses (for example, octulose); deoxy sugars (for example, 2-deoxy-D-ribose, 6-deoxy-L-galactose, and 6-deoxy-L-mannose); amino sugars (for example, D-glucosamine, D-galactosamine, sialic acid, amino uronic acid, and muramic acid); and uronic acids (for example, D-glucuronic acid, D-mannuronic acid, L-guluronic acid, D-galacturonic acid, and L-iduronic acid).

Examples of oligosaccharides include sucrose, guntianose, umbelliferose, lactose, planteose, isolychnoses, α,α-trehalose, raffinose, lychnoses, umbilicin, stachyose, and verbascoses.

Examples of polysaccharides include cellulose, quince seed, starch, galactan, dermatan sulfate, glycogen, gum arabic, heparan sulfate-gum tragacanth, keratan sulfate, chondroitin, xanthan gum, guar gum, dextran, keratosulfate, locust bean gum, and succinoglucan.

Examples of additional polyols include polyoxyethylene methyl glucoside (Glucam E-10) and polyoxypropylene methyl glucoside (Glucam P-10).

Examples of natural water-soluble polymers include plant-based polymers (for example, gum arabic, gum tragacanth, galactan, guar gum, carob gum, gum karaya, carrageenan, pectin, agar, quince seed (quince), algae colloid (cassava extract), starch (rice, corn, potato, and wheat) and glycyrrhizic acid); microbe-based polymers (for example, xanthan gum, dextran, succinoglycan, and pullulan); and animal-based polymers (for example, collagen, casein, albumin, and gelatin).

Examples of semi-synthetic water-soluble polymers include starch-based polymers (for example, carboxymethyl starch and methyl hydroxypropyl starch); cellulose-based polymers (methyl cellulose, ethyl cellulose, methyl hydroxypropyl cellulose, hydroxyethyl cellulose, sodium cellulose sulfate, hydroxypropyl cellulose, carboxymethyl cellulose, sodium carboxymethyl cellulose, crystal cellulose, and cellulose powder); and alginate-based polymers (for example, sodium alginate and propylene glycol alginate).

Examples of synthetic water-soluble polymers include vinyl-based polymers (for example, polyvinyl alcohol, polyvinyl methyl ether, polyvinyl pyrrolidone, and carboxyvinyl polymer); polyoxyethylene-based polymers (for example, Polyethylene Glycol 20,000, 40,000, and 60,000); acrylic polymers (for example, sodium polyacrylate, polyethylacrylate, and polyacrylamide); polyethyleneimine; and cationic polymers.

Examples of humectants include chondroitin sulfate, hyaluronic acid, mucoitin sulfate, trichosanic acid, atelocollagen, cholesteryl-12-hydroxystearate, sodium lactate, bile salt, DL-pyrrolidone carboxylate, short-chain soluble collagen, diglycerin (EO) PO adducts, burr rose extract, yarrow extract, and melilot extract.

Examples of metal-ion sequestrants include 1-hydroxyethane-1,1-diphosphonic acid, 1-hydroxyethane-1,1-diphosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, and trisodium ethylenediamine hydroxyethyl triacetate.

Examples of amino acids include neutral amino acids (for example, threonine and cysteine); and basic amino acids (for example, hydroxylysine). Examples of amino acid derivatives include sodium acyl sarcosine (sodium lauroyl sarcosine), acyl glutamate, sodium acyl β-alanine, and glutathione.

Examples of pH modifiers include buffering agents such as lactic acid-sodium lactate, citric acid-sodium citrate, and succinic acid-sodium succinate.

Examples of water-soluble thickeners include xanthan gum, gum arabic, carrageenan, gum karaya, gum tragacanth, carob gum, quince seed (quince), casein, dextrin, gelatin, sodium pectate, sodium alginate, methyl cellulose, ethyl cellulose, CMC, hydroxyethyl cellulose, hydroxypropyl cellulose, PVA, PVM, PVP, sodium polyacrylate, carboxyvinyl polymer, locust bean gum, guar gum, tamarind gum, dialkyldimethylammonium cellulose sulfate, magnesium aluminum silicate, bentonite, hectorite, Al-Mg silicate (Veegum), laponite, and anhydrous silicic acid.

Examples of water-soluble ultraviolet absorbers include benzophenone-based ultraviolet absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, 2-hydroxy-4-n-octoxybenzophenone, and 4-hydroxy-3-carboxybenzophenone; benzimidazole-based ultraviolet absorbers such as phenylbenzimidazole-5-sulfonic acid and salts thereof, phenylene-bis-benzimidazole-tetrasulfonic acid and salts thereof; 3-(4'-methylbenzylidene)-d,l-camphor, 3-benzylidene-d,l-camphor, urocanic acid, and ethyl urocanate.

Examples of oil-phase components include, but are not limited to, hydrocarbon oils that are generally used in cosmetics and quasi-drugs, higher fatty acids, higher alcohols, synthetic ester oils, silicone oils, liquid oils and fats, solid oils and fats, waxes, and perfumes.

Examples of hydrocarbon oils include isododecane, isohexadecane, isoparaffin, liquid paraffin, ozokerite, squalane, pristane, paraffin, ceresin, squalene, petroleum jelly, and microcrystalline wax.

Examples of higher fatty acids include lauric acid, myristic acid, palmitic acid, stearic acid, behenic acid, oleic acid, undecylenic acid, thioctic acid, isostearic acid, linoleic acid, linolenic acid, eicosapentaenoic acid (EPA), and docosahexaenoic acid (DHA).

Examples of higher alcohols include linear chain alcohols (for example, lauryl alcohol, cetyl alcohol, stearyl alcohol, behenyl alcohol, myristyl alcohol, oleyl alcohol, and cetearyl alcohol); and branched chain alcohols (for example, monostearyl glycerin ether (batyl alcohol)-2-decyltetradecynol, lanolin alcohol, cholesterol, phytosterol, hexyldodecanol, isostearyl alcohol, and octyldodecanol).

Examples of synthetic ester oils include octyl octanoate, nonyl nonanoate, cetyl octanoate, isopropyl myristate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, tripropylene glycol pivalate, diisostearyl malate, glyceryl di-2-heptylundecanoate, glyceryl diisostearate, trimethylolpropane tri-2-ethylhexanoate, trimethylolpropane triisostearate, pentaerythritol tetra-2-ethylhexanoate, glyceryl tri-2-ethylhexanoate, glyceryl trioctanoate, glyceryl triisopalmitate, trimethylolpropane triisostearate, cetyl 2-ethylhexanoate-2-ethylhexyl palmitate, glyceryl trimyristate, tri-2-heptyl undecanoic acid glyceride, castor oil fatty acid methyl ester, oleyl oleate, acetoglyceride, 2-heptylundecyl palmitate, diisobutyl adipate, N-lauroyl-L-glutamate-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, and triethyl citrate.

Examples of silicone oils include chain polysiloxanes (for example, dimethyl polysiloxane, methyl phenyl polysiloxane, and diphenyl polysiloxane), cyclic polysiloxanes (for example, octamethylcyclotetrasiloxane, decamethylcyclopentasiloxane, and dodecamethylcyclohexasiloxane), monovinyl polysiloxanes and divinyl polysiloxanes having a terminal vinyl group, hydrogen siloxanes having a Si-H group, silicon resins that form a three-dimensional network structure, silicone rubber, various modified polysiloxanes (such as amino-modified polysiloxanes, phenyl-modified polysiloxanes, polyether-modified polysiloxanes, alkyl-modified polysiloxanes, and fluorine-modified polysiloxanes), and acrylic silicones.

Examples of oil-soluble ultraviolet absorbers include benzoic acid-based ultraviolet absorbers such as para-aminobenzoic acid (PABA), PABA monoglycerin ester, N,N-dipropoxy PABA ethyl ester, N,N-diethoxy PABA ethyl ester, N,N-dimethyl PABA ethyl ester, and N,N-dimethyl PABA ethyl ester; anthranilic acid-based ultraviolet absorbers such as homomenthyl-N-acetyl anthranilate; salicylic acid-based ultraviolet absorbers such as amyl salicylate, menthyl salicylate, homomenthyl salicylate, octyl salicylate, phenyl salicylate, benzyl salicylate, and p-isopropanol phenyl salicylate; cinnamic acid-based ultraviolet absorbers such as octyl cinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, octyl-p-methoxycinnamate, 2-ethylhexyl-p-methoxycinnamate, 2-ethoxyethyl-p-methoxycinnamate, cyclohexyl-p-methoxycinnamate, ethyl-α-cyano-β-phenylcinnamate, 2-ethylhexyl-α-cyano-β-phenylcinnamate, glyceryl mono-2-ethylhexanoyl-diparamethoxycinnamate, and 3-methyl-4-[methylbis(trimethylsiloxy)silyl]butyl 3,4,5-trimethoxycinnamate; 2-phenyl-5-methylbenzoxazole, 2,2'-hydroxy-5-methylphenyl benzotriazole, 2-(2'-hydroxy-5'-t-octylphenyl)benzotriazole, 2-(2'-hydroxy-5'-methylphenylbenzotriazole, dibenzalazine, dianisoylmethane, 4-methoxy-4'-t-butyldibenzoylmethane, 5-(3,3-dimethyl-2-norbornylidene)-3-pentan-2-one, and octocrylene.

Examples of liquid oils and fats include avocado oil, camellia oil, turtle oil, macadamia nut oil, corn oil, mink oil, olive oil, rapeseed oil, egg yolk oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, *Paulownia fortunei* oil, Japanese paulownia oil, joj oba oil, germ oil, and triglycerin.

Examples of solid oils and fats include cocoa butter, coconut oil, horse tallow, hydrogenated coconut oil, palm oil, beef tallow, mutton tallow, hydrogenated beef tallow, palm kernel oil, lard, beef bone tallow, Japan wax kernel oil, hydrogenated oil, beef leg tallow, Japan wax, and hydrogenated castor oil.

Examples of waxes include beeswax, candelilla wax, cotton wax, carnauba wax, bayberry wax, Chinese wax, whale wax, montan wax, bran wax, lanolin, kapok wax, lanolin acetate, liquid lanolin, sugarcane wax, isopropyl lanolate, hexyl laurate, reduced lanolin, jojoba wax, hard lanolin, shellac wax, POE lanolin alcohol ether, POE lanolin alcohol acetate, POE cholesterol ether, polyethylene glycol lanolate, and POE hydrogenated lanolin alcohol ether.

Examples of perfumes include, but are not limited to, natural perfumes obtained from animals and plants, synthetic perfumes manufactured by chemical synthesis means, and blended perfumes that are mixtures of the above. By blending in a perfume, a cosmetic having excellent fragrance persistence can be obtained.

Examples of perfumes specifically include acetophenone, anisaldehyde, anethole, amyl acetate, amyl salicylate, allyl amyl glycolate, allyl caproate, aldehydes C6 to 20, ambrettolide, ambrettolide, Ambroxan, ionones, Iso E Super, eugenol, aurantiol, galaxolide, calone, coumarin, geraniol, geranyl acetate, sandalore, santalol, sandela, cyclamen aldehyde, cis-3-hexenyl acetate, cis-3-hexenol, citral, citronellyl acetate, citronellol, cineole, dihydromyrcenol, jasmolactone, cinnamic alcohol, cinnamic aldehyde, styralyl acetate, cedryl acetate, cedrol, damascones, damascenones, decalactones, terpinyl acetate, terpineols, tonalid, tonalide, triplal, nerol, bacdanol, vanillin, hydroxycitronellal, phenylethyl acetate, phenylethyl alcohol, hexyl salicylate, vetiveryl acetate, hedione, heliotropin, helional, vertofix, benzyl acetate, benzyl salicylate, benzyl benzoate, pentalid, pentalide, bornyl acetate, myalls, musk ketone, methyl anthranilate, methyl dihydrojasmonate, yara yara, lime oxide, linalyl acetate, linalool, limonene, Lyral, Lilial, rose oxide, Rhodinol, angelica oil, anise oil, almoise oil, basil oil, bay oil, bergamot oil, calamus oil, camphor oil, cananga oil, cardamom oil, cassia oil, cedarwood oil, celery oil, chamomile oil, cinnamon oil, clove oil, coriander oil, cumin oil, dill oil, elemi oil, estragon oil, eucalyptus oil, fennel oil, fenugreek oil, galbanum oil, geranium oil, ginger oil, grapefruit oil, guaiacwood oil, hiba oil, cypress oil, juniper berry oil, lavandin oil, lavender oil, lemon oil, lime oil, mandarin oil, ziram oil, momisa oil, peppermint oil, spearmint oil, mill oil, myrtle oil, nutmeg oil, oakmoss oil, olibanum oil, opoponax oil, orange oil, parsley oil, patchouli oil, pepper oil, perilla oil, petitgrain oil, neroli oil, orange flower oil, pimento oil, allspice oil, pine oil, rose oil, rosemary oil, clary sage oil, sage oil, sandalwood oil, styrax oil, tagetes oil, thyme oil, tuberose oil, valerian oil, vetiver oil, violet leaf oil, wintergreen oil, wormwood oil, ylang-ylang oil, yuzu oil, cassia absolute, genet absolute, hyacinth absolute, immortelle absolute, jasmine absolute, jonquil absolute, narcissus absolute, rose absolute, violet leaf absolute, and benzoin.

Examples of powder components include inorganic powders (for example, talc, kaolin, mica, sericite, muscovite, phlogopite, synthetic mica, red mica, biotite, permiculite, magnesium carbonate, calcium carbonate, aluminum silicate, barium silicate, calcium silicate, magnesium silicate, strontium silicate, tungstate metal salts, magnesium, silica, zeolite, barium sulfate, calcined calcium sulfate (calcined gypsum), calcium phosphate, fluorapatite, hydroxyapatite, ceramic powder, metal soaps (for example, zinc myristate, calcium palmitate, and aluminum stearate), and boron nitride); organic powders (for example, polyamide resin powder (nylon powder), polyethylene powder, polymethyl methacrylate powder, polystyrene powder, styrene-acrylic acid copolymer resin powder, benzoguanamine resin powder, polytetrafluoroethylene powder, and cellulose powder); inorganic white pigments (for example, titanium dioxide and zinc oxide); inorganic red pigments (for example, iron oxide (Bengala) and iron titanate); inorganic brown pigments (for example, γ-iron oxide); inorganic yellow pigments (for example, yellow iron oxide and ocher); inorganic black pigments (for example, black iron oxide and lower titanium oxide); inorganic purple pigments (for example, mango violet and cobalt violet); inorganic green pigments (for example, chromium oxide, chromium hydroxide, and cobalt titanate); inorganic blue pigments (for example, ultramarine and navy blue); pearl pigments (for example, titanium oxide-coated mica, titanium oxide-coated bismuth oxychloride, titanium oxide-coated talc, colored titanium oxide-coated mica, bismuth oxychloride, and argentine); metal powder pigments (for example, aluminum powder and copper powder); organic pigments of zirconium, barium, and aluminum lakes (for example, organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404; Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1); and natural pigments (for example, chlorophyll and β-carotene). Hydrophobized powders obtained by subjecting surfaces of the above powders to hydrophobization treatment are also included.

In the cosmetic of the present invention, other components that are generally used in cosmetics and quasi-drugs, for example, organic amines, polymer emulsions, vitamins, and antioxidants, can be appropriately blended as additional components, within ranges that do not impair the effect of the present invention.

Examples of organic amines include monoethanolamine, diethanolamine, triethanolamine, morpholine, tetrakis(2-hydroxypropyl)ethylenediamine, triisopropanolamine, 2-amino-2-methyl-1,3-propanediol, and 2-amino-2-methyl-1-propanol.

Examples of polymer emulsions include acrylic resin emulsions, polyacrylic acid emulsions, acrylic resin liquids, polyacrylic alkyl ester emulsions, polyvinyl acetate resin emulsions, and natural rubber latex.

Examples of vitamins include vitamins A, B1, B2, B6, C, and E and derivatives thereof, pantothenic acid and derivatives thereof, and biotin.

Examples of additional components that can be blended include preservatives (for example, methylparaben, ethylparaben, butylparaben, and phenoxyethanol); antiphlogistic agents (for example, glycyrrhizic acid derivatives, glycyrrhetinic acid derivatives, salicylic acid derivatives, hinokitiol, zinc oxide, and allantoin); depigmenting agents (for example, placenta extract, *Saxifraga stolonifera* extract, and arbutin); various extracts (for example, cork-tree bark, goldthread rhizome, lithospermum root, Chinese peony root, swertia herb, birch, sage, loquat leaf, ginseng root, aloe, mallow, iris, grape, Job's Tears seed, sponge gourd, lily, saffron, cnidium root, ginger, St. John's wort, restharrow, garlic, chili pepper, aged mikan orange peel, Chinese angelica root, and seaweed); activators (for example, royal jelly, photosensitizers, and cholesterol derivatives); blood circulation promoters (for example, vanillylamide nonylate, nicotinic acid benzyl ester, nicotinic acid β-butoxy ethyl ester, capsaicin, gingerol, cantharides tincture, ichthammol, tannic acid, α-borneol, tocopherol nicotinate, inositol hexanicotinate, cyclandelate, cinnarizine, trazodone, acetylcholine, verapamil, cepharanthine, and γ-oryzanol); anti-seborrheic agents (for example, sulfur and thianthol); and anti-inflammatory agents (for example, tranexamic acid, thiotaurine, and hypotaurine).

Examples of antioxidants include tocopherols, dibutylhydroxytoluene, butylhydroxyanisole, and gallic acid esters. Examples of antioxidant aids include phosphoric acid, citric acid, ascorbic acid, maleic acid, malonic acid, succinic acid, fumaric acid, cephalin, hexametaphosphate, phytic acid, and ethylenediaminetetraacetic acid.

### <Manufacture of cosmetic>

The cosmetic of the present invention can be manufactured by, for example, blending the functional polymer described above or a dried body thereof with other components suited for the desired form of the cosmetic. The manufacturing method of the functional polymer will be described in detail below.

### (Manufacture of functional polymer)

In the present invention, the manufacturing method of the functional polymer of the present invention is not particularly limited. The functional polymer, for example, can be manufactured by any of the following methods (1) to (6).

### Method (1)

A method comprising
a step (i) of crosslinking a temperature-responsive polymer to form a crosslinked network (a) of a crosslinked body of the temperature-responsive polymer, and
a step (ii) of crosslinking a hydrophilic polymer in the presence of the crosslinked network (a) to form an interpenetrating polymer network structure consisting of the crosslinked network (a) and a crosslinked network (b) of a crosslinked body of the hydrophilic polymer.

Wherein, in step (i), a monomer constituting the temperature-responsive polymer may be used in place of the temperature-responsive polymer. In this case, step (i) comprises polymerizing the monomer constituting the temperature-responsive polymer. In step (ii), a monomer constituting the hydrophilic polymer may be used in place of the hydrophilic polymer. In this case, step (ii) comprises polymerizing the monomer constituting the hydrophilic polymer.

### Method (2)

A method comprising
a step (i) of crosslinking a hydrophilic polymer to form a crosslinked network (b) of a crosslinked body of the hydrophilic polymer, and
a step (ii) of crosslinking a temperature-responsive polymer in the presence of the crosslinked network (b) to form an interpenetrating network polymer structure consisting of the crosslinked network (b) and a crosslinked network (a) of a crosslinked body of the temperature-responsive polymer.

Wherein, in step (i), a monomer constituting the hydrophilic polymer may be used in place of the hydrophilic polymer. In this case, step (i) comprises polymerizing the monomer constituting the hydrophilic polymer. In step (ii), a monomer constituting the temperature-responsive polymer may be used in place of the temperature-responsive polymer. In this case, step (ii) comprises polymerizing the monomer constituting the temperature-responsive polymer.

### Method (3)

A method comprising
a step (i) of crosslinking a temperature-responsive polymer to form a crosslinked network (a) of a crosslinked body of the temperature-responsive polymer, and
a step (ii) of dissolving and mixing a hydrophilic polymer (non-crosslinked) in the presence of the crosslinked network (a) to form a semi-interpenetrating polymer network structure consisting of the crosslinked network (a) and the hydrophilic non-crosslinked polymer.

Wherein, in step (i), a monomer constituting the temperature-responsive polymer may be used in place of the temperature-responsive polymer. In this case, step (i) comprises polymerizing the monomer constituting the temperature-responsive polymer. In step (ii), a monomer constituting the hydrophilic polymer may be used in place of the hydrophilic polymer (non-crosslinked). In this case, step (ii) comprises polymerizing the monomer constituting the hydrophilic polymer.

In the present invention, the hydrophilic non-crosslinked polymer may be a linear hydrophilic polymer.

### Method (4)

A method comprising
a step (i) of crosslinking a temperature-responsive polymer in the presence of a hydrophilic non-crosslinked polymer to form a semi-interpenetrating polymer network consisting of the hydrophilic non-crosslinked polymer and a crosslinked network (a) of a crosslinked body of the temperature-responsive polymer.

Wherein, in step (i), a monomer constituting the hydrophilic polymer may be used in place of the hydrophilic polymer (non-crosslinked). In this case, step (i) comprises polymerizing the monomer constituting the hydrophilic polymer. In step (i), a monomer constituting the temperature-responsive polymer may be used in place of the temperature-responsive polymer. In this case, step (i) comprises polymerizing the monomer constituting the temperature-responsive polymer.

### Method (5)

A method comprising
a step (i) of crosslinking a hydrophilic polymer in the presence of a temperature-responsive non-crosslinked polymer to form a semi-interpenetrating polymer network structure consisting of the temperature-responsive non-crosslinked polymer and a crosslinked network (b) of a crosslinked body of the hydrophilic polymer.

Wherein, in step (i), a monomer constituting the temperature-responsive polymer may be used in place of the temperature-responsive polymer (non-crosslinked). In this case, step (i) comprises polymerizing the monomer constituting the temperature-responsive polymer. In step (i), a monomer constituting the hydrophilic polymer may be used in place of the hydrophilic polymer. In this case, step (i) comprises polymerizing the monomer constituting the hydrophilic polymer.

In the present invention, the temperature-responsive non-crosslinked polymer may be a linear temperature-responsive polymer.

### Method (6)

A method comprising
a step (i) of crosslinking a hydrophilic polymer to form a crosslinked network (b) of a crosslinked body of the hydrophilic polymer, and
a step (ii) of dissolving and mixing a temperature-responsive in the presence of the crosslinked network (b) to form a semi-interpenetrating polymer network structure consisting of the crosslinked network (b) and the temperature-responsive non-crosslinked polymer.

Wherein, in step (i), a monomer constituting the hydrophilic polymer may be used in place of the hydrophilic polymer. In this case, step (i) comprises polymerizing the monomer constituting the hydrophilic polymer. In step (ii), a monomer constituting the temperature-responsive polymer may be used in place of the temperature-responsive polymer (non-crosslinked). In this case, step (ii) comprises polymerizing the monomer constituting the temperature-responsive polymer.

In the methods (1) to (6), the polymerization method for polymerizing a monomer is not particularly limited, and radical polymerization, ionic polymerization, polycondensation, or ring-opening polymerization can be suitably used. The catalyst used in the polymerization is appropriately selected according to the monomer, and for example, water, a phosphate buffer solution, a Tris buffer solution, an acetate buffer solution, methanol, or ethanol can be suitably used.

The polymerization initiator is not particularly limited, and for example, persulfates such as ammonium persulfate and sodium persulfate; hydrogen peroxide; peroxides such as t-butyl hydroperoxide and cumene hydroperoxide; azobisisobutyronitrile, and benzoyl peroxide can be suitably used. Among these polymerization initiators, particularly initiators exhibiting oxidizing properties such as persulfates and peroxides can be used as redox initiators with, for example, sodium hydrogen sulfite or N,N,N',N'-tetramethylethylenediamine. Alternatively, light or radiation may be used as an initiator.

The polymerization temperature is not particularly limited, and is generally 5°C to 80°C. The polymerization time is also not particularly limited, and is generally 4 h to 48 h.

The concentrations of the monomer and the crosslinking agent during polymerization need only to be concentrations that yield a temperature-responsive polymer, a hydrophilic polymer, or a crosslinked body thereof, and are not particularly limited. The concentration of the polymerization initiator is also not particularly limited, and is appropriately selected.

In the methods (1) to (6), the method of polymerizing and crosslinking a monomer or crosslinking a polymer (temperature-responsive polymer and/or hydrophilic polymer) to form a crosslinked network of a crosslinked body of the temperature-responsive polymer or the hydrophilic polymer may be a method of polymerizing a monomer in the presence of a crosslinking agent, or may be a method of polymerizing a monomer to form a polymer and then crosslinking the polymer with a crosslinking agent.

In the methods (1) to (6), for example, when using a temperature-responsive non-crosslinked polymer or a hydrophilic non-crosslinked polymer, polymerization conditions or crosslinking conditions are appropriately selected so that no crosslinking is formed between the formed polymers or the crosslinked bodies thereof.

In the methods (1) to (6), the monomer constituting the temperature-responsive polymer, the monomer constituting the hydrophilic polymer, and the crosslinking agent are as described in the sections "Temperature-responsive polymer" and "Hydrophilic polymer" above.

In the methods (1) to (6), the interpenetrating polymer network structure or semi-interpenetrating polymer network structure can be manufactured in a two-stage process of manufacturing a temperature-responsive polymer or a crosslinked body thereof and then, in the presence of the obtained temperature-responsive polymer or the crosslinked body thereof, manufacturing a hydrophilic polymer or a crosslinked body thereof. Alternatively, a temperature-responsive polymer or a crosslinked body thereof and a hydrophilic polymer or a crosslinked body thereof can be manufactured simultaneously in one stage if the polymerization conditions or crosslinking conditions are selected so that crosslinking is not formed between the temperature-responsive polymer or the crosslinked body thereof and the hydrophilic polymer or the crosslinked body thereof. For example, by using a combination of a polymerization method and crosslinking agent for use in the manufacture of the hydrophilic polymer or the crosslinked body thereof and a different polymerization method and crosslinking agent for use in the manufacture of the hydrophilic polymer or the crosslinked body thereof, the functional polymer can be manufactured in a one-stage process.

The dried body of the functional polymer can be manufactured by drying the functional polymer obtained in the manufacturing process of the functional polymer described above.

The method of drying the functional polymer is not particularly limited, and a conventionally known method can be used. Examples of methods of drying the functional polymer can include drying by heating, drying under reduced pressure, spray drying, freeze drying, and solvent substitution.

The functional polymer may be pulverized, as needed, and then blended into the cosmetic of the present invention.

The method of pulverization is not particularly limited. The polymer can be pulverized using, for example, a mechanical pulverizer such as a rotor; a ball mill; or an airflow-type pulverizer, and further classified as needed to obtain particles.

### EXAMPLES

The present invention will be described in further detail with reference to the Examples below. However, the present invention is not limited thereto. Hereinafter, blending amounts are shown in % by mass, unless otherwise specified.

### <<Manufacturing Example 1 Production of functional polymer 1>>

Expert GEL EG412 (INCI; PPG-12/SMDI COPOLYMER, manufactured by PolymerExpert) (hereinafter, also simply referred to as "412") as a temperature-responsive polymer and alginic acid (hereinafter, also simply referred to as "Alg") as a hydrophilic polymer were used to produce a dried body of a functional polymer ("412-Alg") by physical crosslinking.

More specifically, an aqueous solution (1.12 g, 6% by mass) of 412 and a sodium alginate aqueous solution (4.48 g, 6% by mass) were mixed for 2 h, and then precipitated in a 0.25 M calcium chloride aqueous solution (2 L). The precipitate was dried by freeze drying and then pulverized, whereby a dried body of the 412-Alg functional polymer in a white powder form was finally obtained.

### <<Manufacturing Example 2 Production of functional polymer 2>>

412 as the temperature-responsive polymer and hyaluronic acid (hereinafter, also simply referred to as "HA") as the hydrophilic polymer were used to produce a dried body of a functional polymer ("412-HA") by physical crosslinking.

More specifically, a 412 aqueous solution (2.80 g, 6% by mass) and a HA aqueous solution (2.80 g, 6% by mass) were mixed for 2 h, and then precipitated in a 0.25 M calcium chloride aqueous solution (2 L). The precipitate was dried by freeze drying and then pulverized, whereby a dried body of the 412-HA in a white powder form was finally obtained.

### <<Manufacturing Example 3 Production of functional polymer 3>>

Expert GEL EG312 (INCI; PPG-12/SMDI COPOLYMER, manufactured by PolymerExpert) (hereinafter, also simply referred to as "312") as the temperature-responsive polymer and Alg as the hydrophilic polymer were used to produce a dried body of a functional polymer ("312-Alg") by physical crosslinking.

More specifically, a 312 aqueous solution (2.24 g, 6% by mass) and a sodium alginate aqueous solution (3.36 g, 6% by mass) were mixed for 2 h, and then precipitated in a 0.25 M calcium chloride aqueous solution (2 L). The precipitate was dried by freeze drying and then pulverized, whereby a dried body of the 312-Alg in a white powder form was finally obtained.

### <<Manufacturing Example 4 Production of functional polymer 4>>

312 as the temperature-responsive polymer and HA as the hydrophilic polymer were used to produce a dried body of a functional polymer ("312-HA") by physical crosslinking.

More specifically, a 312 aqueous solution (2.80 g, 6% by mass) and a HA aqueous solution (2.80 g, 6% by mass) were mixed for 2 h, and then precipitated in a 0.25 M calcium chloride aqueous solution (2L). The precipitate was dried by freeze drying and then pulverized, whereby a dried body of the 312-HA in a white powder form was finally obtained.

### <<Manufacturing Example 5 Production of functional polymer 5>>

Hydropropyl cellulose (hereinafter, also simply referred to as "HPC") as the temperature-responsive polymer and Alg as the hydrophilic polymer were used to produce a dried body of a functional polymer ("HPC-Alg") by physical crosslinking.

More specifically, a HPC aqueous solution (2.80 g, 6% by mass) and a sodium alginate aqueous solution (2.80 g, 6% by mass) were mixed for 2 h, and then precipitated in a 0.25 M calcium chloride aqueous solution (2 L). The precipitate was dried by freeze drying and then pulverized, whereby a dried body of the HPC-Alg in a white powder form was finally obtained.

### <<Manufacturing Example 6 Production of functional polymer 6>>

HPC as the temperature-responsive polymer and HA as the hydrophilic polymer were used to produce a dried body of a functional polymer ("HPC-HA") by physical crosslinking.

More specifically, a HPC aqueous solution (1.68 g, 6% by mass) and a HA aqueous solution (3.92 g, 6% by mass) were mixed for 2 h, and then precipitated in a 0.25 M calcium chloride aqueous solution (2 L). The precipitate was dried by freeze drying and then pulverized, whereby a dried body of the HPC-HA in a white powder form was finally obtained.

### <<Manufacturing Example 7 Production of functional polymer 7>>

Polyvinyl methyl ether (hereinafter, also simply referred to as "PVME") as the temperature-responsive polymer and Alg as the hydrophilic polymer were used to produce a dried body of a functional polymer ("PVME-Alg") by physical crosslinking.

More specifically, a PVME aqueous solution (2.24 g, 6% by mass) and a sodium alginate aqueous solution (3.36 g, 6% by mass) were mixed for 2 h, and then precipitated in a 0.25 M calcium chloride aqueous solution (2 L). The precipitate was dried by freeze drying and then pulverized, whereby a dried body of the PVME-Alg in a white powder form was finally obtained.

### <<Manufacturing Example 8 Production of functional polymer 8>>

PVME as the temperature-responsive polymer and HA as the hydrophilic polymer were used to produce a dried body of a functional polymer ("PVME-HA") by physical crosslinking.

More specifically, a PVME aqueous solution (1.12 g, 6% by mass) and a HA aqueous solution (4.48 g, 6% by mass) were mixed for 2 h, and then precipitated in a 0.25 M calcium chloride aqueous solution (2 L). The precipitate was dried by freeze drying and then pulverized, whereby a dried body of the PVME-HA in a white powder form was finally obtained.

### <<Manufacturing Example 9 Production of functional polymer 9>>

MebiGel-32 (manufactured by Ichimaru Pharcos Co., Ltd., (butyl acrylate/isopropyl acrylamide/PEG-18 dimethacrylate) cross-polymer; hereinafter, also simply referred to as "MebiGel") as the temperature-responsive polymer and Alg as the hydrophilic polymer were used to produce a dried body of a functional polymer ("MebiGel-Alg") by physical crosslinking.

More specifically, a MebiGel aqueous solution (2.24 g, 6% by mass) and a sodium alginate aqueous solution (3.36 g, 6% by mass) were mixed for 2 h, and then precipitated in a 0.25 M calcium chloride aqueous solution (2 L). The precipitate was dried by freeze drying and then pulverized, whereby a dried body of the MebiGel-Alg in a white powder form was finally obtained.

### <<Manufacturing Example 10 Production of functional polymer 10>>

MebiGel as the temperature-responsive polymer and HA as the hydrophilic polymer were used to produce a dried body of a functional polymer ("MebiGel-HA") by physical crosslinking.

More specifically, a MebiGel aqueous solution (1.68 g, 6% by mass) and a HA aqueous solution (3.92 g, 6% by mass) were mixed for 2 h, and then precipitated in a 0.25 M calcium chloride aqueous solution (2 L). The precipitate was dried by freeze drying and then pulverized, whereby a dried body of the MebiGel-HA in a white powder form was finally obtained.

### <Water absorption performance and water release performance of functional polymers 1 to 10>

The water absorption amount and water release amount were determined as follows for each of the functional polymers 1 to 10 manufactured in the above Manufacturing Examples 1 to 10. The results are shown in Table 1.

### (Water absorption amount)

In a thermostatic box adjusted to room temperature (25°C) at a humidity of 97% RH, 0.1 g of each of the dried bodies of functional polymers manufactured as described above was used to fully absorb water for 48 h to determine the water absorption amount (g/g) by the change in weight before and after water absorption.

### (Water release amount)

In a thermostatic box adjusted to room temperature (25°C) at a humidity of 97% RH, 0.1 g of each of the dried bodies of functional polymers manufactured as described above was allowed to fully absorb water for 48 h, then left to stand for 1.5 h in the thermostatic box at 50°C and 20% RH, and then evaluated for weight change before and after standing to determine the water release amount (g/g).

**[Table 1]**

| (Table 1 Water absorption performance and water release performance of functional polymers 1 to 10) | | | | | | | | | | |
|---|---|---|---|---|---|---|---|---|---|---|
| Manufacturing Example | 1 | 2 | 3 | 4 | 5 | 6 | 7 | 8 | 9 | 10 |
| Composition (wt%) | 412:Alg 2:8 | 412:HA 5:5 | 312:Alg 4:6 | 312:HA 5:5 | HPC:Alg 5:5 | HPC:HA 3:7 | PVME:Alg 4:6 | PVME:HA 2:8 | MebiGel:Alg 4:6 | MebiGel:HA 3:7 |
| Water absorption amount *(g*/*g)* | 1.4 | 1.3 | 1.6 | 1.1 | 1.1 | 0.9 | 1.3 | 1.3 | 1.7 | 1.5 |
| Water release amount (g/g) | 0.5 | 0.6 | 0.7 | 0.7 | 1.0 | 0.6 | 0.6 | 1.0 | 0.6 | 1.0 |

### <<Example 1 and Comparative Example 1 Evaluation of water absorption performance and water release performance of powder cosmetic>>

Based on the raw material components and the blending amounts thereof in Table 2 below, dry pulverization was carried out to produce face powders as the powder cosmetics of Example 1 and Comparative Example 1.

### [Table 2]

**(Table 2)**

| Raw material component | Example 1 | Comparative Example 1 |
|---|---|---|
| | Blending amount (% by mass) | Blending amount (% by mass) |
| Mica | 39 | 42 |
| Hydrophobic mica | 40 | 40 |
| Borosilicate | 5.0 | 5.0 |
| Mica/Silica | 5.0 | 5.0 |
| Mica/Titanium oxide | 1.0 | 1.0 |
| Silica | 5.0 | 5.0 |
| Iron oxide (red, yellow) | 0.5 | 0.5 |
| Functional polymer 5 of Manufacturing Example 5 | 3.0 | (not blended) |
| Chlorphenesin | 0.5 | 0.5 |
| Dimethicone | 1.0 | 1.0 |
| Total | 100 | 100 |

### <Evaluation of water absorption performance>

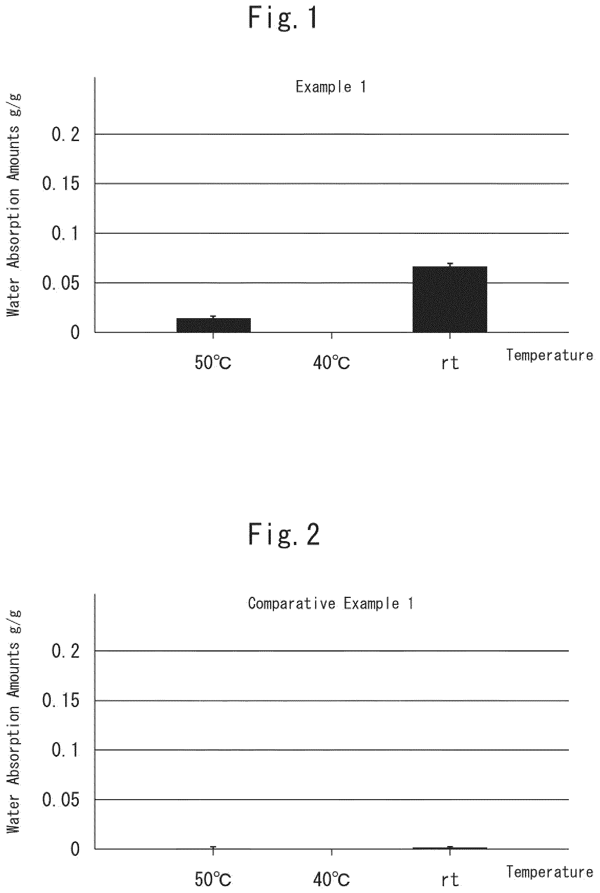

In a thermostatic box adjusted to room temperature (25°C) and 50°C, at a humidity of 55% RH, 0.2 g of each of the face powders of Example 1 and Comparative Example 1 manufactured as described above was used to fully absorb water for 48 h to evaluate water absorption performance by a change in weight before and after water absorption. The evaluation results are shown in FIG. 1 (Example 1) and FIG. 2 (Comparative Example 1). Note that n = 3 (number of independent evaluation experiments carried out was 3).

As is clear from FIG. 1, for the face powder of Example 1, the water absorption amount at room temperature was larger than the water absorption amount at 50°C, i.e., the water absorption performance at room temperature of the face powder of Example 1 was found to be higher than the water absorption performance at 50°C.

From the results of FIG. 1, it was found that the face powder of Example 1 had a water content ratio (first water content ratio) at a temperature (50°C) higher than a predetermined temperature (40°C) that was smaller than a water content ratio (second water content ratio) at a temperature (room temperature) lower than the predetermined temperature. Therefore, it is considered that the HPC-Alg gel of the face powder of Example 1, which had contained water at a relatively low temperature (30°C), can release moisture in an atmosphere having a temperature (50°C) higher than the predetermined temperature (40°C).

As is clear from FIG. 2, the face powder of Comparative Example 1 was not observed to substantially absorb water at room temperature or 50°C, i.e., the water absorption performance thereof was found to be low.

### <Evaluation of water release performance>

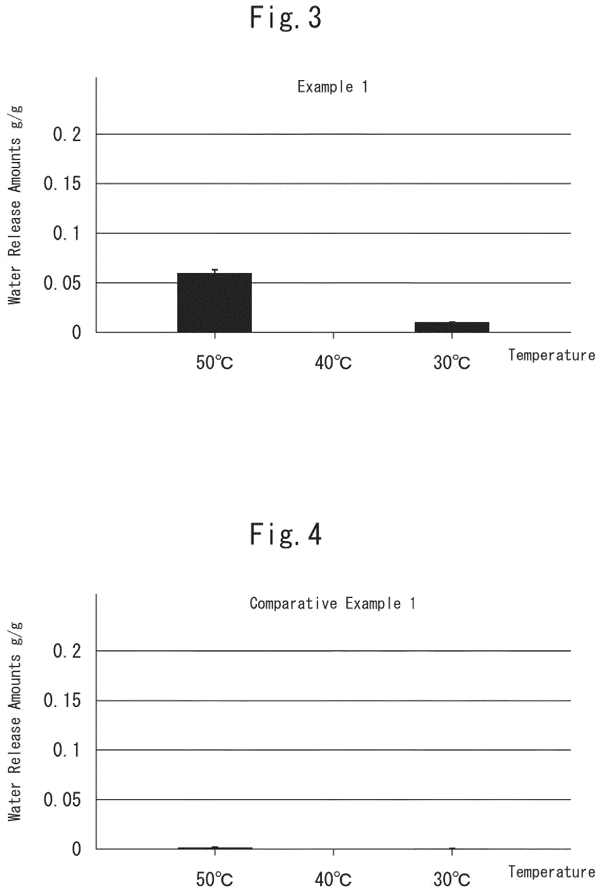

In a thermostatic box adjusted to room temperature (25°C) and a humidity of 55% RH, 0.2 g of each of the dried face powders of Example 1 and Comparative Example 1 was allowed to fully absorb water for 48 h, then left to stand for 1.5 h in a thermostatic box at 30°C and 50°C, at 55%, and then evaluated for weight change before and after standing to evaluate water release performance. The evaluation results are shown in FIG. 3 (Example 1) and FIG. 4 (Comparative Example 1). Note that n = 3.

As is clear from FIG. 3, for the face powder of Example 1, the weight change at 50°C was larger than the weight change at 30°C, i.e., the water release amount at 50°C was found to be larger than the water release amount at 30°C. Therefore, it was found that the face powder of Example 1 had a water content ratio (first water content ratio) at a temperature (50°C) higher than a predetermined temperature (40°C) that was smaller than a water content ratio (second water content ratio) at a temperature (30°C) lower than the predetermined temperature (40°C). In other words, it was suggested that the HPC-Alg gel of the face powder of Example 1, which had contained water at a relative low temperature (30°C), releases water in an atmosphere having a temperature (50°C) higher than the predetermined temperature (40°C).

As is clear from FIG. 4, the face powder of Comparative Example 1 was not observed to have a change in water release amount.

### <<Example 2 Evaluation of water absorption performance and water release performance of liquid cosmetic>>

Based on the raw material components and the blending amounts thereof in Table 3 below, stirring and mixing were carried out to produce lotions as the liquid cosmetic of Example 2.

### [Table 3]

**(Table 3)**

| Raw material component | Example 2 |
|---|---|
| | Blending amount (% by mass) |
| Ion-exchanged water | 78 |
| Glycerin | 5.0 |
| Dipropylene glycol | 9.0 |
| Sorbitol | 1.0 |
| PEG/PPG/Dimethyl ether | 3.0 |
| PPG-decyltetradeceth | 0.1 |
| Functional polymer 5 of Manufacturing Example 5 | 3.0 |
| Citric acid | 0.1 |
| Sodium citrate | 0.1 |
| EDTA-2Na·2H₂O | 0.2 |
| Phenoxyethanol | 0.5 |
| Total | 100 |

### <Evaluation of water absorption performance>

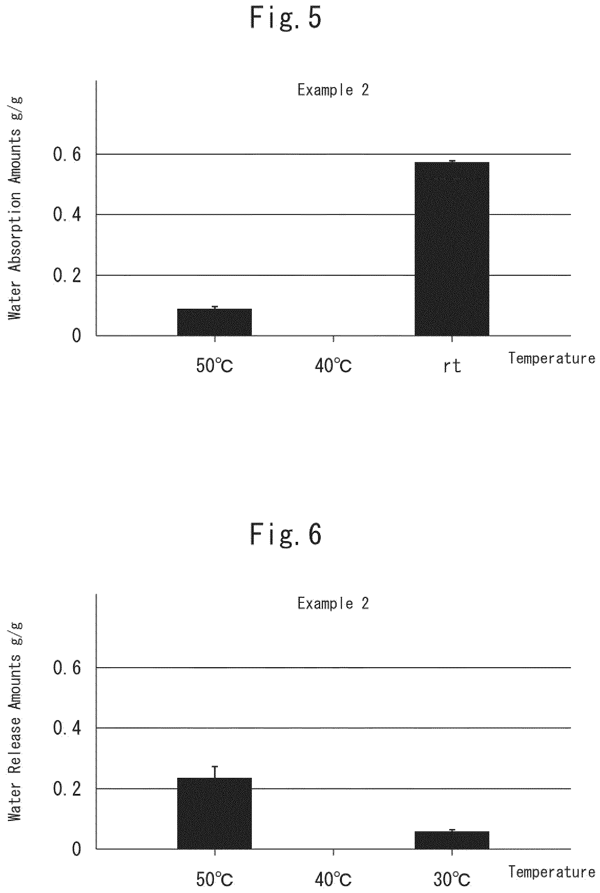

Powders obtained by volatilizing water from each of the lotions of Example 2 were produced. The obtained powders of Example 2 were evaluated for water absorption performance in the same manner as in Example 1 and Comparative Example 1 described above. The evaluation results are shown in FIG. 5 (Example 2). Note than n = 3.

As is clear from FIG. 5, for the powders obtained from the lotions of Example 2, the water absorption amounts at room temperature were larger than the water absorption amounts at 50°C, i.e., the water absorption performance at room temperature of the powders obtained from the lotions of Example 2 was found to be higher than the water absorption performance at 50°C.

### <Evaluation of water release performance>

Powders obtained by volatilizing water from the lotions of Example 2 were produced. The obtained powders of Example 2 were evaluated for water release performance in the same manner as in Example 1 and Comparative Example 1 described above. The evaluation results are shown in FIG. 6 (Example 2). Note that n =3.

As is clear from FIG. 6, for the powders obtained from the lotions of Example 2, weight changes at 50°C were larger than weight changes at 30°C, i.e., the water release amounts at 50°C were found to be larger than the water release amount at 30°C.

### <<Examples 3 and 4 and Comparative Examples 2 and 3>>

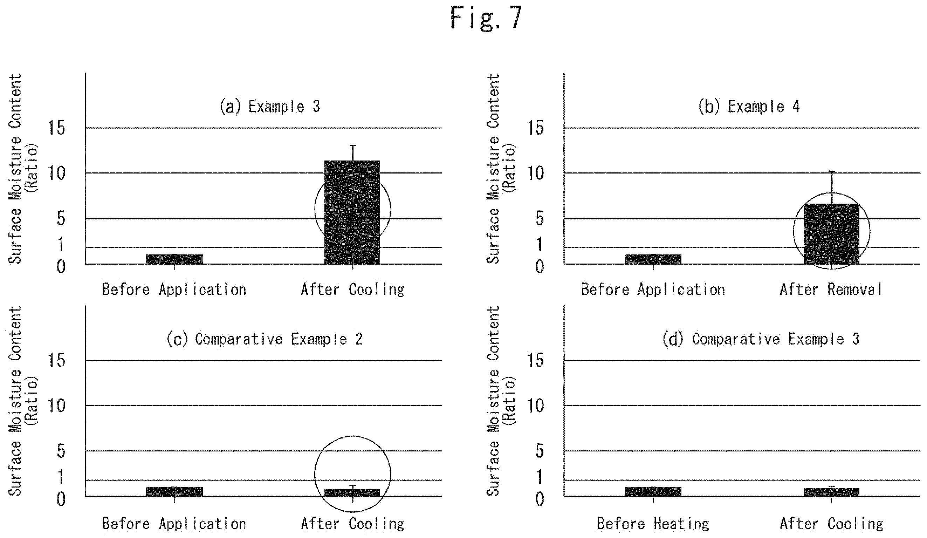

In Example 3, in a thermostatic box adjusted to room temperature and a humidity of 50% RH, the HPC-Alg powder of Manufacturing Example 5 was left to stand for 48 h to produce a water-absorbed HPC-Alg gel. 0.3 g of the HPC-Alg gel was then applied to skin surface, followed by a heating treatment at 40°C for 5 min, and then the HPC-Alg gel was removed. Skin condition after 15 min was measured using a SKICON (SKICON-200EX, manufactured by Yayoi). The surface moisture content of the measured skin is shown in FIG. 7 (a). For ease of comparison, the surface moisture content of the skin before application is also shown (the same applies to the following Example 4 and Comparative Examples 2 and 3).

Example 4, except that the heating and cooling treatments were not carried out after application to the skin surface, was carried out in the same manner as in Example 3. The surface moisture content of the measured skin is shown in FIG. 7 (b).

Comparative Example 2, except that an AlgCa powder (non-temperature-responsive material) was used in place of the HPC-Alg powder, was carried in the same manner as in Example 3. The surface moisture content of the measured skin is shown in FIG. 7 (c).

In Comparative Example 3, the heating and cooling treatments were carried out in the same manner as in Example 3, without applying any gel. The surface moisture content of each of the skin before heating treatment and the skin after cooling treatment was measured, and the results are shown in FIG. 7 (d).

From the results of FIG. 7, the surface moisture content (after cooling) of the skin in the case of Example 3 was found to have a higher value than in the cases of Comparative Examples 2 and 3. Therefore, it was suggested that moisture was released from the HPC-Alg gel of Example 3 and provided to the skin.

The surface moisture content of the skin in the case of Example 3 had a higher value than in the case of Example 4, wherein heating and cooling treatments were not carried out. Therefore, it was suggested that the HPC-Alg gel of Example 3, under heating, releases more moisture in an atmosphere having a temperature higher than the predetermined temperature.

### <<Example 5 and Comparative Example 4>>

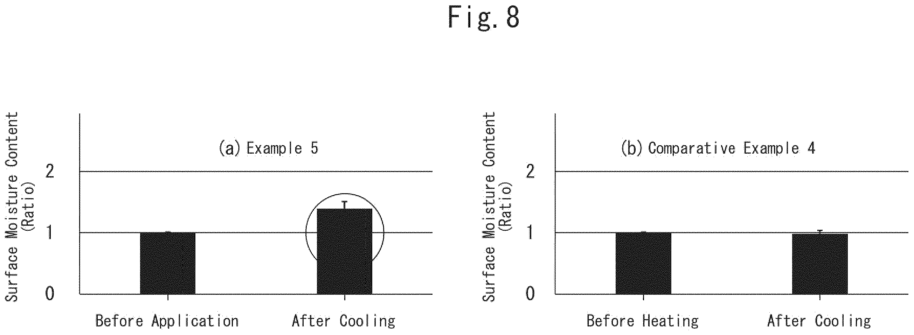

In Example 5, in a thermostatic box adjusted to room temperature and a humidity of 50% RH, the MebiGel-Alg powder of Manufacturing Example 9 was left to stand for 48 h to produce a water-absorbed MebiGel-Alg gel. 0.3 g of the MebiGel-Alg gel was then applied to skin surface, followed by a heating treatment at 40°C for 5 min, and then the MebiGel-Alg gel was removed. Skin condition after 15 min was measured using a SKICON (SKICON-200EX, manufactured by Yayoi). The surface moisture content of the measured skin is shown in FIG. 8 (a).

In Comparative Example 4, the heating and cooling treatments were carried out in the same manner as in Example 5, without applying any gel. The surface moisture contents of the skin before the heating treatment and the skin after the cooling treatment were each measured and are shown in FIG. 8 (b).

From the results of FIG. 8, the surface moisture content (after cooling) of the skin in Example 5 was found to have a higher value than in the case of Comparative Example 4. This suggested that moisture was released from the MebiGel-Alg gel of Example 5 and provided to the skin.

## Claims

1. A cosmetic comprising a functional polymer in which a temperature-responsive polymer and a hydrophilic polymer form an interpenetrating polymer network structure or a semi-interpenetrating polymer network structure.

2. The cosmetic according to claim 1, wherein the functional polymer has a first water content ratio at a temperature higher than a predetermined temperature that is smaller than a second water content ratio at a temperature lower than the predetermined temperature, and the functional polymer thereby releases, in an atmosphere having a temperature higher than the predetermined temperature, moisture absorbed in an atmosphere having a temperature lower than the predetermined temperature.

3. The cosmetic according to claim 2, wherein the predetermined temperature is any temperature in a range of 5 to 60°C.

4. The cosmetic according to claim 1 or 2, wherein the temperature-responsive polymer is at least one polymer selected from the group consisting of poly(N-alkyl (meth)acrylamide); poly(N-vinylalkylamide); poly(N-vinylpyrrolidone); poly(N-vinylcaprolactam); poly(2-alkyl-2-oxazoline); polyvinyl alkyl ether; polyethylene oxide and polypropylene oxide and copolymers thereof; copolymers of glycols such as polyethylene glycol and polypropylene glycol and diisocyanates such as hexamethylene diisocyanate, diphenylmethane diisocyanate, toluene diisocyanate, and methylene diphenyl diisocyanate; copolymers composed of components selected from alkyl (meth)acrylates such as methyl (meth)acrylate, ethyl (meth)acrylate, and butyl (meth)acrylate, acrylamides such as N-phenyl acrylamide, isopropyl acrylamide, and N-tert-butyl acrylamide, and di(meth)acrylic acids such as 1,3-butandiol dimethacrylate, 1,6-hexanediol dimethacrylate, and PEG di(meth)acrylate; poly(oxyethylene vinyl ether); cellulose derivatives; polyurethane derivatives; and copolymers comprising these, or a crosslinked body thereof.

5. The cosmetic according to claim 1 or 2, wherein the hydrophilic polymer is at least one polymer selected from the group consisting of alginic acid, hyaluronic acid, carrageenan, xanthan gum, glucomannan, tara gum, locust bean gum, guar gum, fenugreek gum, chitosan, cellulose derivatives, poly(meth)acrylic acid, polyethylene glycol, and copolymers thereof, or a crosslinked body thereof.

6. The cosmetic according to claim 1 or 2, which is a powder cosmetic.

7. The cosmetic according to claim 1 or 2, which is a liquid cosmetic.
